# EUROPEAN PATENT APPLICATION

(11) **EP 2 400 299 A1**
(43) Date of publication of application: **28.12.2011**
(21) Application number: 10162358.5
(22) Date of filing: 07.05.2010
(51) Int. Cl.: G01N 33/50, C12Q 1/68

(54) **Novel factors that promote triplet repeat expansions**

(71) Applicant: National University of Ireland, Galway, Galway (IE)
(72) Inventor: Lahue, Robert, Moycullen Co. Galway (IE); Debacker, Kim, Moycullen Co. Galway (IE); Kirkham, Lucy, Bushypark Co. Galway (IE)
(74) Representative: Gates, Marie Christina Esther

(57) **Abstract**

A pharmaceutical composition comprising an agent that inhibits the protein target of the human genes listed in Figure 4. A method of identifying a therapeutic agent for the treatment of a disease(s) caused by expansions of DNA trinucleotide repeats and a method of identifying a gene involved in DNA trinucleotide expansion.

## Description

### Field of the Invention

The present invention relates to genes which, when disrupted, lead to lower rates of triplet repeat expansions. The invention also relates to use of these gents to identify therapeutic agents which can disrupt somatic expansions of triplet repeats. Such drugs would find use in slowing the progression of neurological disorders caused by expansions i.e. gain of length, in DNA trinucleotide repeats. Such diseases or disorders include Huntington's disease, myotonic dystrophy type I, Fragile X syndrome types A and E, Spinocerebellar ataxia (SCA) types 1, 2, 3, 6, 7, 12, 17; Spinal and bulbar muscular atrophy; dentatorubral-pallidoluysian atrophy; and Friedreich's ataxia.

### Background to the Invention

Patients afflicted with disorders caused by expansions in DNA trinucleotide repeats, inherit an expanded trinucleotide repeat from their patents, the inherited allele largely determining whether they will get the disease. In addition, patients suffer ongoing expansions in key target organs such as brain and muscle depending on the disease, and these ongoing somatic expansions are thought to determine the progression of the disease.

Somatic expansions occur by complex genetic mechanisms, and there may be more than one mechanism, depending on the disease gene and target organ. Normal cellular proteins appear to act aberrantly at DNA trinucleotide repeats to promote (accelerate) their expansion.

### Object of the Invention

It is thus an object of the invention to provide compositions for and methods of preventing somatic expansions, thus reducing disease progression and the suffering of patients. A further object is to provide additional numbers of and types of potential genes involved in somatic expansions, in part to address the possibility that multiple genetic mechanism may underlie somatic expansions. A further object is to discover new promoting factors and deduce how they accelerate expansions in budding yeast and human cells. HDAC inhibitors are in clinical trials to alleviate symptoms of Huntington's disease¹ and other diseases caused by triplet repeat expansions². Our results suggest that HDAC inhibitors may have a second, beneficial effect of suppressing somatic expansions and thus slowing progression of the disease.

### Summary of the Invention

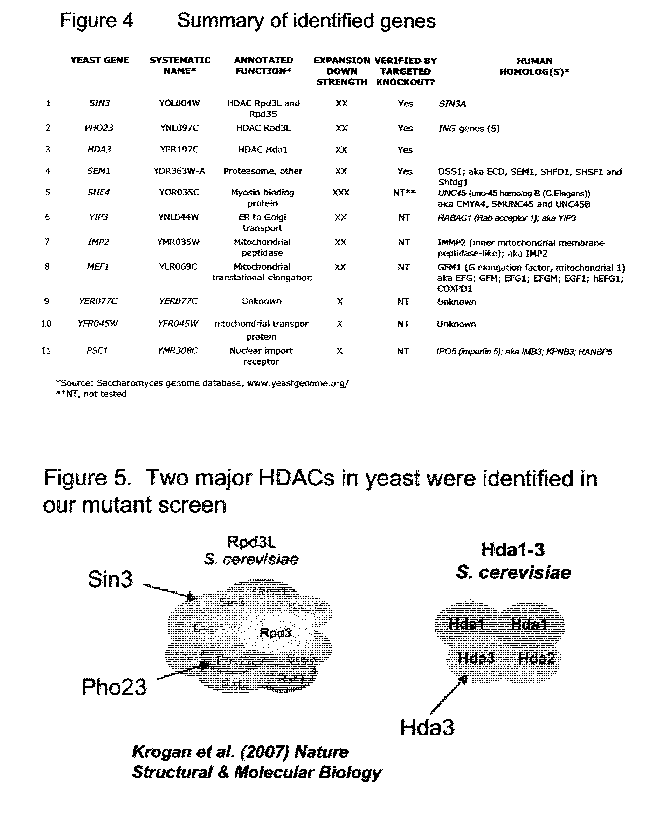

The present invention relates to pharmaceutical composition comprising an agent that inhibits the protein target of the human genes identified in Figure 4, together with a pharmaceutically acceptable carrier.

Another aspect of the invention is a method of identifying a therapeutic agent for the treatment of diseases caused by expansions of DNA trinucleotide repeats comprising providing a cell culture, the cells expressing at least one of the human genes identified in Figure 4, incubating the cells with a potential therapeutic agent, and determining the expression of the at least one gene, a reduced level of expression of the at least one gene when compared with a control culture to which no potential therapeutic agent has been added, being indicative of a therapeutic agent. Preferably the cell culture is a human cell culture.

Suitably the human genes may be selected from the group comprising SIN 3A, ING genes or DSS1 (also known as SEM1, SHFD1, SHSF1 and Shfdg1).

A method of treating diseases caused by expansions of DNA trinucleotide repeats comprising administering to a patient in need of such treatment, an agent that inhibits the protein target of the human genes identified in Figure 4. The agent may be a small molecule which interferes with protein-protein interactions, an inhibiting antibody or a small interfering RNA.

The invention also relates to use of a yeast gene as identified in Figure 4 to identify homologous human genes which can be used to identify therapeutic agents for the treatment of diseases caused by expansions of DNA trinucleotide repeats in the methods as describe above. This is possible because of the conservation of genes between humans and laboratory yeasts.

Suitably the yeast genes may be selected from the group comprising SIN 3, PHO 23, HAD 3 and SEM1.

The invention also provides a panel of genes for use in identifying therapeutic agents for the treatment of diseases caused by expansions of DNA trinucleotide repeats comprising the human or yeast genes identified in Figure 4.

Also provided is a method of identifying genes involved in DNA trinucleotide expansion comprising inserting a reporter gene downstream of a CTG repeat in the yeast genome, mutagenising the yeast cells and identifying down mutants of the reporter gene.

The invention also relates to a method of gene therapy comprising disrupting expression of at least one of the human genes identified in Figure 4, in somatic body cells.

In another aspect the invention provides a method of identifying a gene involved in DNA trinucleotide expansion comprising;-
(a) inserting a reporter gene into a yeast genome, the reporter gene having a trinucleotide repeat inserted into its promoter,
(b) mutatgenising a yeast genomic library,
(c) transforming the mutated yeast sequences into the yeast genome of step (a),
(d) identifying mutated gene(s) with a reduced expansion rate compared to wild type, based on an increase in expression of said reporter gene.

In a particuar embodiment the method may comprise:-
(a) inserting the reporter gene CAN-1 or URA3 into a yeast genome, the reporter gene having a trinucleotide repeat CTG inserted into its promoter, wherein inhibition of expression of the CAN-1 or URA3 reporter gene imparts resistance to canavanine or 5-fluoroorotic acid respectively,
(b) mutatgenising a yeast genomic library,
(c) transforming the mutated yeast sequences into the yeast genome of step (a),
(d) identifying mutated gene(s) with a reduced expansion rate compared to wild type, based on an increase in sensitivity of the transformed yeast to canavanine or 5-fluoroorotic acid.

The invention also relates to a method of gene therapy comprising disrupting expression of at least one of the human genes identified in Figure 4, in somatic body cells and to the use of a yeast gene as identified in Figure 4 to identify homologous human genes which can be used to identify therapeutic agents for the treatment of diseases caused by expansions of DNA trinucleotide repeats.

### Detailed Description of the Drawings

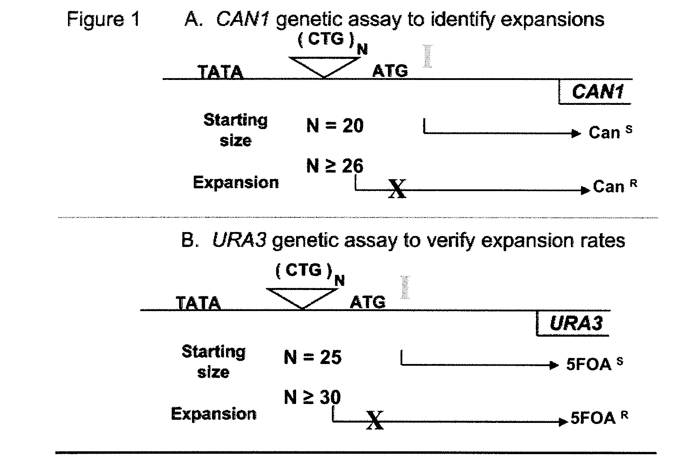

**Figure 1**. Genetic assays in yeast to monitor triplet repeat expansions.
   Panel A. The length of the CTG repeat governs expression of the reporter gene, *CAN 1.* The starting repeat length of 20 allows transcriptional initiation to occur such that *CAN1* is transcribed and translated in-frame. Expression of *CAN1* makes the cell sensitive to the presence in the growth media of the drug canavanine (Cans). Cells expressing *CAN1* die on canavanine-containing media. If an expansion occurs first, and adds 6 repeats or more (to a final length of ≥ 26 CTG repeats), transcription is predicted to initiate farther upstream. These transcripts will now include an out-of-frame ATG codon; any translation of these transcripts will occur out-of-frame with respect to *CAN1,* thus precluding production of the Can1 protein. Cells with an expanded CTG repeat can survive on media containing canavanine (Can^{R}). The number of Can^{R} cells in the population is proportional to expansion rate.
   Panel B. The same logic from Panel A applies here. There are two differences. The readout gene is *URA3* (1), whose expression makes the cells sensitive to 5-fluoroorotic acid (5FOA^{S}). Expansions lead to drug resistance (5FOA^{R}). *URA3* readout is included in this system to guard against any gene-specific anomalies associated with *CAN1.* The second difference from Panel A is that the starting repeat length is slightly longer, 25 repeats, and that 30 repeats or more must be attained to generate drug resistance.

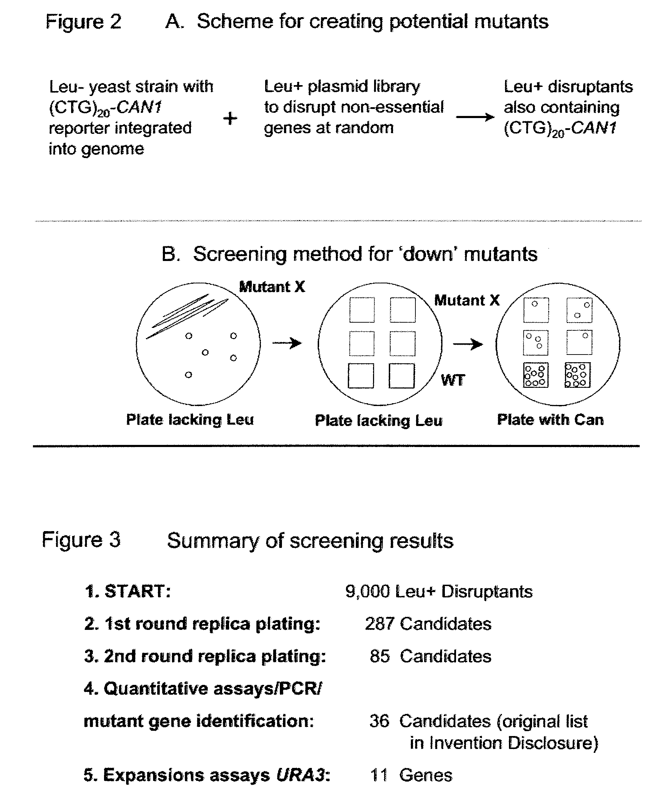

**Figure 2**. Schematic of mutant screening
   Panel A shows the general approach used to find down mutants. The starting yeast strain BL1466 *(MATa leu2-3 leu2-112 his3-Δ trp1-289 ura3-52 L1can1::kanMX)* included the *HIS3*-marked *CAN1* reporter from Fig. 1A integrated at the *LYS2* locus. This strain was mutagenized at random with a plasmid library that causes disruption of non-essential yeast genes (2). Disruptants were identified based on the ability to grow without leucine in the growth medium (Leu⁺).
   Panel B demonstrates how down mutants were identified. Leu+ disruptants (left) were grown in rectangular patches (center), and then replica plated to medium containing canavanine (right). On medium with canavanine, only cells with expansions survive. The number of survivors is proportional to the expansion rate. A 'down' mutant has a lower expansion rate and therefore fewer survivors.
**Figure 3**. Summary of screening process
**Figure 4**. Summary of 11 genes identified in the screen showing detailed analysis of the behavior of genes 1-4 *(SIN3, PHO23, HDA3,* and *SEMI).*
**Figure 5**. Schematic diagram showing the HDAC complexes in yeast that contain the proteins encoded by *SIN3, PHO23,* and *HDA3,* respectively.
**Figure 6**. Expansion rate measurements in targeted knockouts. Expansion rates using *CAN1* readout were performed in the BL1466 background (see legend to Fig. 2). For assays with *URA3* readout, the related strain BL1370, *MATa leu2-3 leu2-112 his3-* Δ *trp1-289 ura3-52* with the *HIS3*-marked reporter integrated at *LYS2 was used.*
   Following identification of the mutants as described in Figs. 3 and 4, targeted knockouts were made in the original starting strain. This is to help validate that the genes identified in the screen are the proper targets for further investigation. In all cases, this verification held good. The expansion rates are shown in these targeted mutants. Some targeted double mutants were examined. For the *sem1* mutant, strains where the wild type yeast gene was added back on a low copy plasmid *(pSEM1)* were tested. In addition, *DSS1* was also tested by add-back *(pDSS1),* where *DSS1* is the human homolog of yeast *SEM1.*

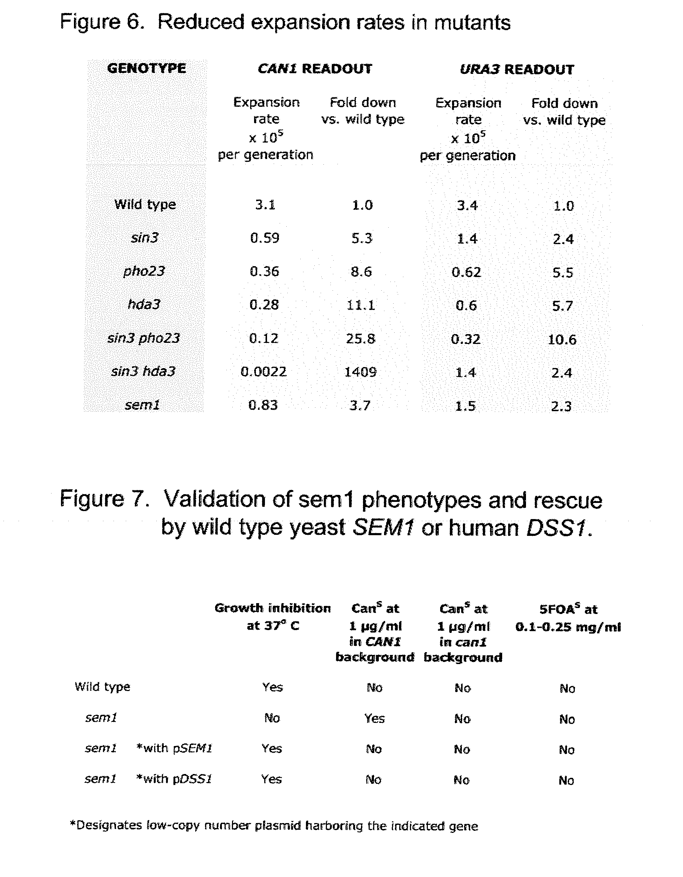

**Figure 7****.** Validation of phenotypes associate with targeted knockout of yeast *SEM1.*

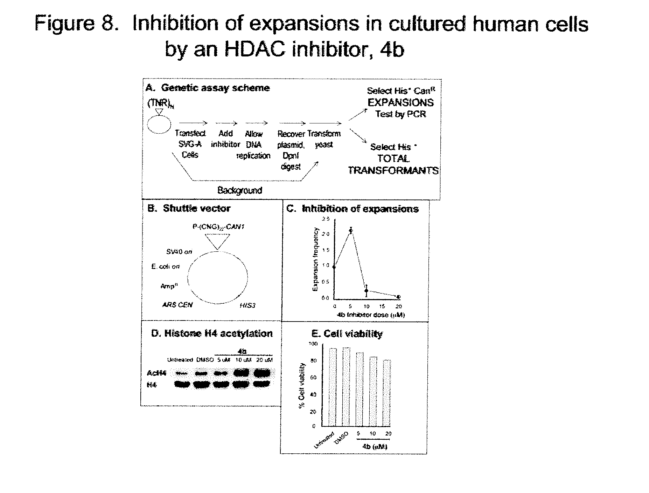

**Figure 8****.** Evidence that inhibition of HDACs in human cell culture also inhibits expansions
   Panel A shows how expansions are measured in human tissue culture cells (9). In this experiment, we included treatment of the cells with the HDAC inhibitor 4b.
   Panel B, details of the shuttle vector containing the trinucleotide repeat.
   Panel C, expansion frequency in response to treatment with increasing amounts of 4b. Results from two independent experiments
   Panel D, analysis of 4b action, as judged by accumulation of acetylated histone H4 (AcH4), relative to total H4 in an immunoblot.
   Panel E, cell viability measured by nigrosin staining. Results from two experiments.

### Detailed Description of the Invention

### Materials and Methods

*Yeast strains and plasmids.* The yeast strain BL1466 *(MATa leu2-3 leu2-112 his3-Δ trp1-289 ura3-52 Δcan1::kanMX)* included the *HIS3*-marked (CTG)20-*CANI* reporter integrated at the *LYS2* locus. Reporter targeting was achieved by restriction cleavage with Bsu36I. In some cases, the reporter was cleaved with StuI to target its integration at *URA3* Correct reporter integration was confirmed by Southern blotting (11). For *URA3* expansion readout, the strain used was BL1370, *MATa leu2-3 leu2-112 his3- Δ trp1-289 ura3-52* with the *HIS3*-marked (CTG)25-*URA3* reporter integrated at *LYS2* (1). For *sem1* rescue studies, the wild type yeast *SEMI* or wild type human *DSS1* genes were cloned into vector pRS314, followed by transformation into the *sem1* background strain (BL1466 plus *sem1).*

*Screening with disruption library.* To identify novel proteins involved in promoting CTG repeat expansions, a random screen of yeast insertion mutants was performed. A plasmid disruption library (2) was used to disrupt random genes in yeast strain BL1455. A high throughput replica plating method was used to identify mutants with decreased rates of expansions, based on reduced number of papillae when transferred to media containing canavanine. Mutants with decreased rates of contractions as compared to wild type were further analyzed by fluctuation analysis. Several mutants showed decreased rates of CTG repeat expansions; mutant genes were identified by Vectorette PCR as described in (3).

*Quantitative expansion assays.* Expansion rates were measured by fluctuation analysis as described previously (1,11) and as shown in Fig. 1. Mutation rates were calculated by the method of the median (12). Single-colony PCR analysis of expansions was performed as previously described (1,13), and rates were corrected by multiplying the percent bona fide expansions by the apparent mutation rates obtained by fluctuation analysis (11).

*Expansion assays in human cells.* Expansions were assayed by minor modification of a published protocol (9). The CTG repeat was 22 in length, and the reporter gene was *CAN1.* SVG-A cells were transfected with the vector using lipofectamine 2000 (Invitrogen). Parallel cultures of cells were treated with inhibitor 4b, with DMSO as a vehicle-only control, or left untreated. Cell viability was assessed by nigrosin staining and microscopic visualization two days after treatment. Two days after transfection, the plasmid was isolated as described (9) and expansions were measured as Can^{R} yeast transformants, and the observed frequency was normalized to total plasmid population size, as determined by number of ampicillin-resistant *E. coli* obtained by transformation. Expansions were verified by PCR and the final expansion frequency was calculated as (observed frequency) x (fraction of PCR-verified expansions). Immunoblot analysis was performed on acid extracts of cells (Abcam), using antibodies against histone H4 (Abcam 31830) and acetylated histone H4 (Upstate 06-866).

### RESULTS

**Rationale and identification of target genes**. If a protein, Protein X, helps promote trinucleotide repeat (TNR) expansions, then mutation of its gene, Gene X, should lead to fewer expansions. Such mutants are referred to herein as 'down' mutants. The approach was to use the budding yeast *Saccharomyces cerevisiae* to look for down mutants. The human homolog(s) of these yeast genes would therefore be potential targets for drug inhibition to reduce somatic expansions in human cells and potentially in patients.

To monitor expansions, the starting yeast strain BL1466 contains a reporter gene system with a CTG repeat inserted into its promoter (Fig. 1). Two versions of this assay, using different readout genes, were employed to guard against anomalies specific to one or the other reporter gene. Fig. 1A describes the use *of CAN1* as the first reporter gene. At the starting CTG length of 20 repeats, the reporter gene *CAN1* is expressed, which makes the cells sensitive to the drug canavanine. If an expansion occurs to add 6 or more repeats (final length of 26 repeats or larger), *CAN1* is not expressed (Fig. 1A) and the cells are resistant to canavanine. The number of canavanine-resistant cells in the population is directly related to expansion rate. PCR analysis of the canavanine-resistant cells showed that approx 80-90% are authentic expansions. The *TNR-CAN1* reporter was used to screen for mutants, and to provide quantitative readout of expansion rates in the targeted mutant strain. The second reporter gene was *URA3* (Fig. 1B). As we published earlier (1), expansions of the CTG repeat from starting length of 25 to final lengths of 30 or more repeats can be monitored by resistance to the drug 5-fluoroorotic acid (5FOA). We showed previously that typically 90% or more 5FOA-resistant yeast in this assay harbor expansions, as verified by PCR (1). The TNR-*URA3* reporter was used to validate the down expansion phenotype during the screening process and in the targeted mutant strains.

The starting yeast strain, with *TNR-CAN1* reporter integrated into its genome, was mutagenized using a gene disruption library (2). This library creates disruption of yeast genes at random in the genome. Only the 4,800 non-essential genes can be tested, however, since disruption of an essential gene kills the cell. Successful disruption causes the cells to become Leu+ (Fig. 2A). Approximately 9,000 Leu+ colonies were analyzed subsequently for a 'down' phenotype; this is estimated to cover approximately 50% of the non-essential genes in the genome. TNR expansion rates were estimated using a qualitative screening process (Fig. 2B). Disruptants were sought that gave fewer canavanine-resistant papillae (outgrowth colonies) than wild type, suggesting a lower rate of expansions. 287 candidates were identified (Fig. 3), and these were subjected to a second round of screening, to help weed out false positives. 85 candidates passed the second round, and then quantitative assessments were performed to estimate expansion rates. 36 candidates emerged. These 36 were then evaluated for 'down' expansion phenotype using a variant of the genetic assay, where the readout gene for expansion is *URA3.* 11 mutants also gave a down phenotype with the *URA3* system (Fig. 3). The mutated gene in each mutant was identified using PCR and DNA sequencing (described in (3). These 11 mutants are the focus of this application. **Target gene identities and subsequent validation**. The 11 genes arising from this work are shown in Fig. 4. The first four genes, *SIN3, PHO23, HDA3,* and *SEMI* have undergone detailed analysis, as described below. Fig. 5 shows that *SIN3, PHO23,* and *HDA3* encode subunits of the histone deacetylase complexes Rpd3L and Hdal, respectively.

Several common experiments were performed to analyze *SIN3, PHO23, HDA3,* and *SEM1.* In each case, the gene identification was confirmed using targeted knockout of each gene in the original, unmutated yeast strains BL1466 and BL1370. The purpose of this experiment is to verify that the expansion down phenotype is conferred by the candidate gene. In every case, this was found to be true. Figure 6 shows that each single mutant *(sin3, pho23, hda3,* and *sem1)* reduced expansion rates for both the *CAN1* readout system and when *URA3* was the reporter gene. Expansion rates were reduced by 2.3-fold to 11.1- fold (Fig. 6). Thus the targeted knockouts confirmed the identity of the genes found in the screen.

A second common experiment was to evaluate whether the mutated genes conferred any changes to sensitivity/resistance to canavanine or 5FOA. In other words, in the absence of an expansion reporter, do any of the mutated genes change the cell's sensitivity to either drug? Each mutant strain was tested for growth ability on media containing either canavanine (30 µg/ml) or 5FOA (0.5 mg/ml), and compared to the wild type control. For *sin3, pho23,* and *hda3,* there was no detectable change in growth rates compared to control. Thus these mutations do not alter the cell's response to the drugs. The results for *sem1* are described in more detail later.

*Double mutant analysis.* For some combinations of genes, double mutant analysis was performed to look for genetic interactios. The *sin3 pho23* double mutant, which inactivates two subunits of the Rpd3L HDAC (Fig. 5), showed expansion rate decreases of 10.6- to 25.8-fold, compared to wild type (Fig. 6). When both Rpd3L and Hda1 were inactivated in the *sin3 hda3* double mutant, two different responses were seen (Fig. 6). For the *URA3* readout system, the rate decrease was 2.4-fold, similar to either single mutant. However, expansions measured by the *CAN1* readout were inhibited by approx 1,400-fold compared to wild type. While the magnitude of the *CAN1* phenotype does not extend to the *URA3* system, it does indicate that some double mutants have the capacity to nearly eliminate expansions.

*Re-location of the TNR reporter to a different genomic integration site.* For some mutants, there is expansion rate data available when the (CTG)20-*CANI* reporter was integrated at the *URA3* locus on chromosome V, instead of the *LYS2* locus on chromosome II. This experiment tests whether the down phenotype applies to more than one genomic locus. Preliminary results show down mutant phenotypes of 2-fold or more decrease in expansion rates for *sin3, pho23, sin3 pho23,* and *pho23 hda3.* The *hda3* single mutant showed an expansion rate similar to wild type, although it must be stressed that this is a preliminary result. The other mutants have yet to be tested in this experiment.

*SEM1.* Mutants *of SEMI* were previously reported to be unable to grow at 37° and to be sensitive to 1 µg/ml canavanine in a *CAN1* background (4,5). Our original *sem1* mutant and the targeted *sem1* deletion (Fig. 7) both showed these phenotypes, consistent with correct gene identification. Moreover, we were able to reverse these phenotypes by adding-back on a plasmid either the wild type yeast *SEMI* gene or the human homolog *DSS1* (Fig. 7). Thus, either the yeast gene *SEMI* or the human gene *DSS1* can correct the defect of *a sem1* mutant. None of the strains tested were sensitive to 1 µg/ml canavanine when the genome harbored a *can1* mutant. Similarly, no sensitivity to 5FOA was detected (Fig. 7). These latter two observations rule out the possibility that low expansion rates, detected by resistance to canavanine or 5FOA, could be due to strain anomalies.

*Human homologs of the yeast genes.* The gene identification in Fig. 4 shows the human homologs that we could identify for the 11 yeast genes, based on database information. Eight of the 11 genes have identifiable human homologs. As shown above, the human *DSS1* gene can rescue some defects of the yeast *sem1* mutant (Fig. 7). Analysis was also carried out for class I human HDACs, homologous to yeast Rpd3L (Figs. 4 and 5). We tested whether expansions are inhibited in cultured human astrocytes (6) by a synthetic compound called 4b, an inhibitor of human Class I HDACs. Joel Gottesfeld, a chemist at The Scripps Research Institute, La Jolla, California, USA, provided the inhibitor. 4b selectively targets the HDAC3 isotype (7), although high concentrations also inhibit HDAC1. Human HDACs 1 and 3 are homologs to yeast Rpd3L (8). Figure 8, Panel A illustrates that a shuttle vector containing a TNR is transfected into SVG-A astrocytes. Expansions are scored by recovering the plasmid and using yeast as a biosensor to select expansions that occurred in the SVG-A cells. We published this assay (9); the new wrinkle is use of 4b. Panel B provides details of the shuttle vector. Panel C shows the response to 4b dose. At 10-20 µM inhibitor, expansions are suppressed by 4- to 10-fold. Care must be taken with dose, however, as low inhibitor dose (5 µM, Panel C) actually stimulates expansions, presumably due to indirect effects. Results are from two experiments. Panel D shows the expected accumulation of acetylated H4 upon 4b treatment. Panel E indicates good viability of 4b-treated cells (≥84% of DMSO-only controls, based on nigrosin staining in two experiments). Thus suppression of expansions in 4b-treated cells cannot be attributed to cell loss. Overall this experiment indicates that HDAC inhibition in human cells has the potential to suppress expansions, and that HDAC3 and/or HDAC1 are potential targets. **Identifying additional targets in yeast**. *Robotic screen.* The yeast screen described above yielded approx 50% coverage of the 4,800 or so non-essential genes of budding yeast. This analysiscan be extended to 100% of these genes by exploiting the yeast haploid deletion set (10). The deletion set is a collection of circa 4,800 strains, each with a targeted disruption of a known gene. By introducting our (CTG)25-*URA3* reporter into each strain, we can screen for additional down mutants, using the scheme in Fig. 2B and scoring 5FOA resistance. One advantage of this screen is that the 11 mutants that we already identified (Fig. 4) will be present in the deletion set. They should therefore be detected as down mutants, thereby providing internal controls to validate the screening method.

Our recent analysis: (1) confirms the original finding of the mutant screen; (2) shows that double HDAC mutants eliminate nearly all expansions in yeast; and (3) finds that the mutant effect can be phenocopied when wild type cells are treated with an HDAC inhibitor. These results not only help refine the science; they also support the reason for testing expansion effects in human tissue culture cells or in transgenic mice treated with drugs with suitable HDAC inhibitory effects.

### Conclusion

We found that budding yeast histone deacetylase complexes (HDACs) Rpd3L and Hda1 are new promoting factors for somatic expansions, based on the fact that expansions are suppressed in Rpd3L and Hda1 mutants.

Our working hypothesis, the 'TNR chromatin' model, is that histone deacetylation at the TNR chromatin controls access of replication and repair proteins to the TNR DNA. We favor this cis-acting model because it helps explain the site-specific nature of expansions. Also, it is consistent with evidence from the literature, such as the profound integration site dependence for TNR expansions in transgenic mouse models (e.g. ref. 29). There are two alternative, *tran*s-acting possibilities. The 'indirect expression' model suggests that histone acetylation controls expression of some distant gene or genes, which in turn act in *trans* at the TNR to modulate expansions. A second *trans-*acting model, 'non-histone acetylation', invokes that Rpd3L and Hda1 deacetylate some unknown, non-histone protein(s) which then act to promote expansions. While the three models are not necessarily mutually exclusive, we hypothesize that the TNR chromatin model is the major mechanism.

The words "comprises/comprising" and the words "having/including" when used herein with reference to the present invention are used to specify the presence of stated features, integers, steps or components but does not preclude the presence or addition of one or more other features, integers, steps, components or groups thereof.

It is appreciated that certain features of the invention, which are, for clarity, described in the context of separate embodiments, may also be provided in combination in a single embodiment. Conversely, various features of the invention which are, for brevity, described in the context of a single embodiment, may also be provided separately or in any suitable sub-combination.

### Literature cited

1. Rolfsmeier, M. L., Dixon, M. J., Pessoa-Brandao, L., Pelletier, R., Miret, J. J., and Lahue, R. S. (2001) Genetics 157, 1569-1579
2. Burns, N., Grimwade, B., Ross-Macdonald, P. B., Choi, E.-Y., Finberg, K., Roeder, G. S., and Snyder, M. (1994) Genes Dev. 8, 1087-1105
3. Bhattacharyya, S., Rolfsmeier, M. L., Dixon, M. J., Wagoner, K., and Lahue, R. S. (2002) Genetics 162, 579-589
4. Funakoshi, M., Li, X., Velichutina, I., Hochstrasser, M., and Kobayashi, H. (2004) J. Cell. Sci. 117, 6447-7457
5. Sone, T., Saeki, Y., Toh-e, A., and Yokosawa, H. (2004) J. Biol. Chem. 279, 28807-28816
6. Major, E. O., Miller, A. E., Mourrain, P., Traub, R. G., de Widt, E., and Sever, J. (1985) Proc. Natl. Acad. Sci. U. S. A. 82, 1257-1261
7. Herman, D., Jenssen, K., Burnett, R., Soragni, E., Perlman, S. L., and Gottesfeld, J. M. (2006) Nat. Chem. Biol. 2, 551-558
8. Yang, X.-J., and Seto, E. (2008) Nat. Rev. Mol. Cell. Biol. 9, 206-218
9. Claassen, D. A., and Lahue, R. S. (2007) Hum. Mol. Genet. 16, 3088-3096
10. Tong, A. H. Y., Evangelista, M., Parsons, A. B., Xu, H., Bader, G. D., Page, N., Robinson, M., Raghibizadeh, S., Hogue, C. W. V., Bussey, H., Andrews, B., Tyers, M., and Boone, C. (2001) Science 294, 2364-2368
11. Dixon, M. J., Bhattacharyya, S., and Lahue, R. S. (2004) Methods Mol. Biol. 277, 29-45
12. Lea, D. E., and Coulson, C. A. (1948) J. Genet. 49, 264-284
13. Miret, J. J., Pessoa-Brandao, L., and Lahue, R. S. (1998) Proc. Natl. Acad. Sci. U S. A. 95, 12438-12443

## Claims

1. A pharmaceutical composition comprising an agent that inhibits the protein target of the human genes identified in Figure 4, together with a pharmaceutically acceptable carrier.

2. A method of identifying a therapeutic agent for the treatment of diseases caused by expansions of DNA trinucleotide repeats comprising providing a cell culture, the cells expressing at least one of the human genes identified in Figure 4, incubating the cells with a potential therapeutic agent, and determining the expression of the at least one gene, a reduced level of expression of the at least one gene when compared with a control culture to which no potential therapeutic agent has been added, being indicative of a therapeutic agent.

3. A method as claimed in claim 2 wherein the cell culture is a human cell culture.

4. A method as claimed in claim 2 or claim 3 wherein the human genes are selected from the group comprising SIN 3A, ING genes or DSS1 (also known as SEM1, SHFD1, SHSF1 and Shfdg1).

5. A method of treating diseases caused by expansions of DNA trinucleotide repeats comprising administering to a patient in need of such treatment, an agent that inhibits the protein target of the human genes identified in Figure 4.

6. A method as claimed in claim 4 wherein the yeast genes are selected from the group comprising SIN 3, PHO 23, HAD 3 and SEM1.

7. A panel of genes for use in identifying therapeutic agents for the treatment of diseases caused by expansions of DNA trinucleotide repeats comprising the human or yeast genes identified in Figure 4.

8. A method of identifying a gene involved in DNA trinucleotide expansion comprising;-
(a) inserting a reporter gene into a yeast genome, the reporter gene having a trinucleotide repeat inserted into its promoter,
(b) mutatgenising a yeast genomic library,
(c) transforming the mutated yeast sequences into the yeast genome of step (a),
(d) identifying mutated gene(s) with a reduced expansion rate compared to wild type, based on an increase in expression of said reporter gene.

9. A method of identifying a gene involved in DNA trinucleotide expansion as claimed in claim 8 comprising:-
(a) inserting the reporter gene CAN-1 or URA3 into a yeast genome, the reporter gene having a trinucleotide repeat CTG inserted into its promoter, wherein inhibition of expression of the CAN-1 or URA3 reporter gene imparts resistance to canavanine or 5-fluoroorotic acid respectively,
(b) mutatgenising a yeast genomic library,
(c) transforming the mutated yeast sequences into the yeast genome of step (a),
(d) identifying mutated gene(s) with a reduced expansion rate compared to wild type, based on an increase in sensitivity of the transformed yeast to canavanine or 5-fluoroorotic acid.

10. A method of gene therapy comprising disrupting expression of at least one of the human genes identified in Figure 4, in somatic body cells.

11. The use of a yeast gene as identified in Figure 4 to identify homologous human genes which can be used to identify therapeutic agents for the treatment of diseases caused by expansions of DNA trinucleotide repeats.
